# EUROPEAN PATENT SPECIFICATION

(11) **EP 4 536 185 B1**
(45) Date of publication and mention of the grant of the patent: **18.11.2026**
(21) Application number: 23730515.6
(22) Date of filing: 05.06.2023
(51) Int. Cl.: A61K 9/00, A61K 9/08, A61K 9/19, A61K 31/519, A61K 47/02, A61P 35/00, A61K 33/14

(54) **STABLE PHARMACEUTICAL COMPOSITIONS COMPRISING 5,10-METHYLENE-(6R)-TETRAHYDROFOLIC ACID AND NACL**
STABILE PHARMAZEUTISCHE ZUSAMMENSETZUNGEN MIT 5,10-METHYLEN-(6R)-TETRAHYDROFOLSÄURE UND NACL
COMPOSITIONS PHARMACEUTIQUES STABLES COMPRENANT DE L'ACIDE 5,10-MÉTHYLÈNE-(6R)-TÉTRAHYDROFOLIQUE ET NACL

(30) Priority: 08.06.2022 EP 22177928
(43) Date of publication of application: 16.04.2025
(73) Proprietor: Merck Patent GmbH, 64293 Darmstadt (DE)
(72) Inventor: MOSER, Rudolf, 6460 ALTDORF / URI (CH); GROEHN, Viola, 6460 ALTDORF / URI (CH); AMMANN, Thomas, 6460 ALTDORF / URI (CH)
(74) Representative: Merck Patent Association
(86) International application number: PCT/EP2023/064968
(87) International publication number: WO 2023/237480

(56) References cited:
- CA-A1- 2 477 101
- US-A- 5 455 236
- US-B2- 10 570 134
- US-B2- 6 995 158

## Description

The present invention is directed towards stable lyophilisates comprising 5,10-methylene-(6R)-tetrahydrofolic acid and NaCl, wherein the molar ratio of 5,10-methylene-(6R)-tetrahydrofolic acid to NaCl is from 1:1 to 1:10, preferably from 1:5 to 1:10, more preferably 1:7.

As used herein, 5,10-CH₂-(6R)-THF refers to 5,10-methylenetetrahydrofolic acid in its naturally occurring isomeric form (N-[4-[(6aR)-3-amino-1,2,5,6,6a,7-hexahydro-1-oxoimidazo[1,5-f]pteridin-8(9H)-yl]benzoyl]-L-glutamic acid), wherein the chiral centres at C6 of the pteridine ring and the α-carbon of the glutamic acid moiety are in their naturally occurring configuration.

### BACKGROUND OF THE INVENTION

5,10-methylenetetrahydrofolic acid is known as a medicament used in combination with 5-fluorouracil (5-FU) in the treatment of solid tumours (Seley, K. L. Drugs 4 (1), 99, 2001). The active form, 5,10-CH₂-(6R)-THF, achieves its chemotherapeutic effect together with the base analogue and 5-FU metabolite 5-FdUMP by inhibiting the enzyme thymidylate synthase (TS). TS catalyses the conversion of deoxyuridylate (dUMP) to deoxythymidylate (dTMP), which is an essential building block for DNA synthesis. Deactivation of TS occurs by formation of a covalent, ternary inhibition complex between TS, the base analogue 5-FdUMP, and 5,10-CH₂-(6R)-THF, often referred to as inhibitory ternary complex. An enhancement of the cytotoxic effect of 5-FU can be achieved by increasing the intracellular concentration of 5,10-CH₂-(6R)-THF, whereupon the stability of the ternary complex is increased. This causes direct inhibition of DNA synthesis and repair, which ultimately results in cell death and delay of tumor growth.

However, there are undesirable properties associated with 5,10-CH₂-(6R)-THF that limit its pharmaceutical use. For example, 5,10-CH₂-(6R)-THF is highly susceptible to oxidation and chemical degradation that results in unfavourably high impurity levels. Susceptibility to oxidation and chemical degradation of 5,10-CH₂-(6R)-THF is especially high when the compound is present in its amorphous form, having a large surface e.g. in its pharmaceutical use form as a lyophilisate or in re-dissolved form such as solutions for injection. It is well known that to be amenable for pharmaceutical use the respective composition needs to fulfil several requirements including high stability, such that effective storage over an acceptable period of time can be achieved, without exhibiting a significant change in the composition's physicochemical characteristics, ease of handling and processing, etc.. 5,10-methylenetetrahydrofolic acid is an addition product of tetrahydrofolic acid and formaldehyde (see e.g. Poe, M. et al. Biochemistry 18 (24), 5527, 1979; Kallen, R. G. Methods in Enzymology 18B, 705, 1971) and is known for its extremely high sensitivity to oxidation by air as well as instability in neutral and/or acidic environments potentially leading to chemical degradation and/or hydrolysis (see e.g. Odin, E. et al., Cancer Investigation 16 (7), 447, 1998; Osborn, M. J. et al., J. Am. Chem. Soc. 82, 4921, 1960; Hawkes, J., and Villota, R. Food Sci. Nutr. 28, 439, 1989). Attempts to stabilize compositions of 5,10-methylenetetrahydrofolates included e.g. (i) rigorous exclusion of atmospheric oxygen by the use of special technical devices for the reconstitution of solid formulations and the injection of 5,10-methylenetetrahydrofolates in an air-free environment (see e.g. Odin, E. et al., Cancer Investigation 16 (7), 447, 1998; U.S. Pat. No. 4,564,054); (ii) addition of a reducing agent such as L(+)-ascorbic acid or salts thereof, reduced gamma-glutathione, beta-mercaptoethanol, thioglycerol, N-acetyl-L-cysteine, etc. as an antioxidant for the highly sensitive 5,10-methylenetetrahydrofolic acid and for tetrahydrofolic acid in particular; (iii) stabilization by means of cyclodextrin inclusion compounds (see e.g. EP 0 579 996); (iv) addition of citrate while adjusting the pH to a basic value (see e.g. EP 1 641 460); or (v) the addition of dicarboxylic acids (see e.g. EP 18752161.2); or (vi) formation of various crystalline forms such as the sulfate salts (see e.g. EP 0 537 492) or hemisulfate salts (see e.g. EP 2 837 631).

From a clinical perspective the availability of stable forms, especially lyophilisates and solutions of 5,10-CH2-(6R)-THF having a high content of the active ingredient and being free of any kind of stabilisers would be an advantage. Citric acid, for example, has been linked to various undesired effects like e.g. QT_{c} elongation (Laspina et al. Transfusion 42 (2002) p.899, Toyoshima et al. Clinical Nutrition (2006) 25, 653-660), inducing hypocalcaemia (Payne et. Al. J. Physiol. (1964), 170, pp. 613-620), etc..

The teachings of the prior art does not readily allow the preparation of such stable stabiliser-free compositions of 5,10-CH2-(6R)-THF, and thus there still remains a great need for stable liquid and solid-state pharmaceutical compositions, especially lyophilisates, of 5,10-CH2-(6R)-THF, which do not contain extraneous stabilising compounds such as stabilisers, buffers, reducing agents and the like. It is a derived problem that the stabilised versions of 5,10-CH2-(6R)-THF known in the prior art usually contain less than 50% of the active drug compound due to the dilution in the final dosage form of the drug by the stabilising additives.

There still remains a great need for stable pharmaceutical compositions, especially lyophilisates, having a high content of 5,10-CH₂-(6R)-THF.

### SUMMARY OF THE INVENTION

It has now surprisingly been found that certain solid compositions comprising 5,10-methylene-(6R)-tetrahydrofolic acid and NaCl overcome the previously discussed known drawbacks and allow for the preparation of pharmaceutical compositions of high purity, high stability, high content of 5,10-methylene-(6R)-tetrahydrofolic acid, and a low content of either oxidation products or other chemical degradation products. The advantageous stability characteristics of the solid compositions of the present invention will allow their effective use in medicinal applications.

The addition of NaCl to the lyophilisation solution of 5,10-CH₂-(6R)-THF has surprisingly been found to maintain the purity of the active ingredient 5,10-CH₂-(6R)-THF in the lyophilisate at a remarkably high level and at the same time maintain the amounts of by-products at an acceptable low level. The obtained lyophilized products contain a high content of 5,10-CH₂-(6R)-THF and exhibit a stability over months or more without significant loss of active ingredient, i.e. the amount of active ingredient is maintained at or above 95% and more preferably at or above 98% for several months, including most preferably about 99%, 99.5% or 99.8% even without having added any stabiliser, buffer, reducing agent and the like. This enables the manufacturing, storage and use of lyophilisates of 5,10-CH₂-(6R)-THF without significant decomposition before reconstitution.

It has further been found that lyophilisates of the present invention have a different water content than the corresponding active ingredient and the previously known lyophilisates of 5,10-CH₂-(6R)-THF, e.g. the ones containing citrate while adjusting the pH to a basic value.

Lyophilisation or freeze-drying is a dehydration process that works by freezing an aqueous solution containing a dissolved material therein and then reducing the surrounding pressure to allow the frozen water to sublimate directly from the solid phase to the gas phase. There are usually four stages in a complete lyophilisation process: pretreatment, freezing, primary drying, and secondary drying.

Pretreatment includes any method of treating the material prior to freezing. This may include the addition of other components. Pretreatment is possible but not necessary in the preparation of stable lyophilisates comprising 5,10-CH₂-(6R)-THF and NaCl.

Freezing is often done by placing an aqueous solution of the material in a freeze-drying flask which is cooled by mechanical refrigeration, or by using dry ice or liquid nitrogen. On a larger scale, freezing the aqueous solution is usually done using a freeze-drying machine. In this step, it is important to cool the material below its triple point, the lowest temperature at which the solid and liquid phases of the material can coexist. This ensures that sublimation rather than melting will occur in the following steps. Freezing is preferably done at temperatures of -45°C to -70°C in the preparation of stable lyophilisates comprising 5,10-CH₂-(6R)-THF and NaCl.

Annealing for 1 to 2 hours at shelf temperatures around -5°C to -2°C is possible but not necessary in the preparation of stable lyophilisates comprising 5,10-CH₂-(6R)-THF and NaCl.

During the primary drying phase, the pressure is lowered (to the range of a few millibars), and enough heat is supplied to the material for the ice to sublimate. In this initial drying phase, about 95% of the water in the material is sublimated. This phase may be slow (can be several days in the industry), because, if too much heat is added, the material's structure could be altered. In the primary drying phase, pressure is controlled through the application of partial vacuum. The vacuum speeds up the sublimation, making it useful as a deliberate drying process. In the preparation of stable lyophilisates comprising 5,10-CH₂-(6R)-THF and NaCl, the primary drying phase is started at a freezing temperature of preferably between -45°C to -70°C. Then during the primary drying phase, the temperature is, after an optional starting period of preferably 10 to 120 minutes at freezing temperature, increased over time to preferably about 0°C. During the primary drying phase, a pressure of preferably about 50 µbar to 200 µbar is held.

The secondary drying phase aims to remove unfrozen water molecules, since the ice was removed in the primary drying phase. In this phase, the temperature is raised higher than in the primary drying phase, and can even be above 0°C, to break any physico-chemical interactions that have formed between the water molecules and the frozen material. Usually, the pressure is also lowered in this stage to encourage desorption (typically in the range of microbars, or fractions of a pascal). Secondary drying is preferably done at temperatures up to about 25°C to 30°C and a pressure of about 50 µbar to 200 µbar in the preparation of stable lyophilisates comprising 5,10-CH₂-(6R)-THF and NaCl.

The primary and secondary drying phases may be combined by following a temperature ramp from freezing temperature to temperatures up to about 25°C to 30°C and a pressure ramp from about 50 µbar to 200 µbar in the preparation of stable lyophilisates comprising 5,10-CH₂-(6R)-THF and NaCl. The temperature ramp may contain multiple holding steps where the temperature is kept constant for some time. Preferably the holding steps, if any, are at freezing temperature, at about 0°C and at about 25°C to 30°C.

After the lyophilisation process is complete, the vacuum is usually broken with an inert gas, such as nitrogen, before the material is sealed. At the end of the operation, the final residual water content of lyophilisates comprising 5,10-CH₂-(6R)-THF and NaCl is usually below 5%, preferably at most 3%, even more preferably at most 2% and most preferably below 1%. In a particularly preferred embodiment, the lyophilisates comprising 5,10-CH₂-(6R)-THF and NaCl are essentially anhydrous.

Stability is a critical property and component of pharmaceutical formulations as well as in studies of the same and during drug development. Chemical stability studies are performed both in solution and solid state. It is an established fact that solution state and solid-state stability can differ both qualitatively and quantitatively. Also, in solid state the stability of a crystalline material and an amorphous material, such as a lyophilisate can differ. Extensive studies are performed for chemical stability of a drug substance and pharmaceutical compositions thereof by exposing it to variety of stressors, like high temperature and/or high humidity. These studies also provide information on the degradation products and help in developing meaningful specifications as well as the intrinsic stability of the pharmaceutical composition. Most common pathways for drug degradation include i.a. hydrolysis, oxidation, and photochemical degradation.

The purpose of stability testing is to provide evidence on how the quality of a product varies with time under the influence of a variety of environmental factors such as temperature, humidity, and light, and to establish a suitable shelf life for the pharmaceutical product and recommended storage conditions in order to ensure patient safety.

One embodiment of the present invention is directed to stable lyophilisates comprising 5,10-methylene-(6R)-tetrahydrofolic acid (5,10-CH₂-(6R)-THF) and NaCl, wherein the molar ratio of 5,10-methylene-(6R)-tetrahydrofolic acid to NaCl is from 1:1 to 1:10.

In a preferred embodiment, the lyophilisate comprises 5,10-CH₂-(6R)-THF and NaCl and no further chemotherapeutic agents. In a further preferred embodiment, the employed 5,10-CH₂-(6R)-THF has a purity of greater than 99%, and more preferably a purity of greater than 99.5%.

One preferred embodiment is directed to a lyophilisate comprising 5,10-CH₂-(6R)-THF and NaCl, further characterized in containing one or more stabilising agents such as stabilisers, buffers, reducing agents and the like, as defined hereinbelow.

One preferred embodiment is directed to a lyophilisate comprising 5,10-CH₂-(6R)-THF and NaCl, further characterized in containing neither citric acid nor citrate ions.

Lyophilisates of the present invention are substantially amorphous solid compositions having an enhanced stability, such as an enhanced storage stability.

Lyophilisates of the present invention have a high content of 5,10-CH₂-(6R)-THF, typically at least 25% w/w, such as at least 30% w/w, such as at least 35% w/w, such as at least 40% w/w.

Lyophilisates of the present invention may further preferably be reconstituted into an aqueous pharmaceutical formulation to be administered into a patient in need thereof.

A further aspect is directed to a process for the preparation of the lyophilisates of the present invention which comprises the steps of
(i) dissolving 5,10-methylene-(6R)-tetrahydrofolic acid, or a salt thereof, in a solvent or solvent system;
(ii) adding NaCl, and optionally excipients for improving cake formation and/or to adjust the osmolality of the solution;
(iii) freezing the solution; and
(iv) thereafter removing the frozen solvent under vacuum;
   or
(i) dissolving 5,10-methylene-(6R)-tetrahydrofolic acid, or a salt thereof, in a solvent or solvent system;
(iia) adding NaOH to raise the pH to about 11 and optionally adding excipients for improving cake formation and/or to adjust the osmolality of the solution;
(iib) adding HCl to lower to pH to about 9.3 and optionally adding excipients for improving cake formation and/or to adjust the osmolality of the solution;
(iii) freezing the solution; and
(iv) thereafter removing the frozen solvent under vacuum.

The solution of step (ii), (ii)a or (iib) can optionally be filtered through a sterile filter, before step (iii) is performed.

Preferably, the solvent is water or an aqueous solvent system.

Preferably, the pH of the solution after step (ii) resp. (iib) is above 6, more preferred about 8-14, even more preferably about 9.3.

In the steps (i), (ii), (iia), or (iib) optionally excipients for improving cake formation and/or to adjust the osmolality of the solution and/or matrix stabilisers may be added. Such optional excipients may be one or more bulking agents selected from arginine, glycine, histidine, dextran and/or polyethylene glycol.

The structure and porosity of the lyophilized cake is important, since good pore formation may facilitate drying and transport of the water during the drying cycle. Also, electrolytes or sodium chloride may be added to the composition to adjust the osmolality. Osmolality adjustment can be done before or after freeze drying. The reconstituted (re-dissolved) lyophilisate solution preferably has an osmolality in the range of 250 - 350 mOsm. However, an osmolality of 200 - 600 mOsm can be tolerated as well and will depend on the volume to be administered as well as the injection/infusion time.

A further aspect is directed to reconstituted pharmaceutical compositions of the lyophilisates of the present invention comprising 5,10-CH₂-(6R)-THF and NaCl and a pharmaceutically acceptable carrier or diluent, such as sterile water or a liquid pharmaceutically acceptable vehicle, optionally further comprising excipients for improving cake formation and/or to adjust the osmolality of the solution and/or matrix stabilisers.

A further aspect is directed to reconstituted pharmaceutical compositions of the lyophilisates of the present invention comprising 5,10-CH₂-(6R)-THF and NaCl and a pharmaceutically acceptable carrier or diluent, such as sterile water or a liquid pharmaceutically acceptable vehicle, optionally further comprising excipients for improving cake formation and/or to adjust the osmolality of the solution and/or matrix stabilisers, and which reconstituted pharmaceutical compositions optionally further comprise at least one additional therapeutic agent including but not limited to, bactericides, antibiotics, antivirals, antiseptics, antineoplastics, anticancer compounds such as chemotherapeutic agents, antifungals, and/or anti-inflammatory agents or other bioactive or therapeutic agents that are suitable for human use, in particular anticancer compounds such as chemotherapeutic agents, for example 5-FU and derivatives, and antifolates, e.g. methotrexate, Pemetrexed.

In the present text, the term "liquid pharmaceutically acceptable vehicle" refers to propylene glycol, a polyethylene glycol, ethanol, dimethyl sulfoxide (DMSO), N-methylpyrrolidinone (NMP), glycofurol, isopropylidene glycerol (Solketal), glycerol formal, acetone, tetrahydrofurfuryl alcohol, monoglyme, diglyme, dimethyl isosorbide or ethyl lactate, mixtures thereof, or aqueous mixtures thereof.

In the present text, the term "stabilisers" or "stabilising agents" refers to buffers such as citrate (or citric acid and salts thereof), dicarboxylates such as succinate, malate and maleate, TRIS; N-tris(hydroxymethyl)methyl-2-aminoethanesulfonic acid, (TES); 3-(N-morpholino) propanesulfonic acid (MOPS) ; N,N-bis(2-hydroxyethyl)-2-aminoethane-sulfonic acid (BES); MES; MOPSO; HEPES; phosphate; carbonate; ammonium ; mono-, di- and tri-alkylammonium; mono-, di- and tri-hydroxyl-alkylammonium; glutamate; borate; lactate, as well as combinations of these. The term "stabilisers" or "stabilising agents" further relates to reducing agents such as L-(+) ascorbic acid or salts thereof, reduced γ-glutathione, β-mercaptoethanol, thioglycerol, N-acetyl-L-cysteine, etc. which may act as an antioxidant for the sensitive 5,10-methylenetetrahydrofolic acid, and for the tetrahydrofolic acid in particular.

In the present text, the term "solvent" relates to solvents which may be used in freeze drying processes. Similarly, the term "solvent system", as used herein, relates to mixtures of solvents which may be used in freeze drying processes.

"Solutions" as referred to in the present invention, comprise aqueous solutions as well as solutions in organic solvents.

Typically, "aqueous solutions" mean solutions in water, saline, water containing small amounts of buffers, water containing isotonic amounts of NaCl, or mixtures of water with organic solvents. Typical organic solvents include DMSO, acetonitrile, acetone, methanol, or ethanol.

A further aspect is directed to the use the reconstituted pharmaceutical compositions in therapy, preferably in cancer chemotherapy.

Further aspects are directed at the use of the stable lyophilisates, including reconstituted solutions thereof, in combination with at least one or more additional therapeutic agents. Preferred additional therapeutic agents comprise in this context chemotherapeutic agents and other anti-cancer drugs. Particularly preferred drugs, which may be combined with the pharmaceutical compositions of the present invention, comprise fluorinated nucleic acids such as 5-fluorouracil or prodrugs or analogues thereof.

The reconstituted pharmaceutical compositions may be used in therapy, specifically in cancer chemotherapy, i.e. in a method for treatment of cancer, which comprises administering a therapeutically effective amount of 5,10-CH₂-(6R)-THF to a subject in need of such treatment.

Another embodiment is directed at the use of the reconstituted pharmaceutical compositions in therapy, specifically in cancer chemotherapy, which comprises administering a therapeutically effective amount of 5,10-CH₂-(6R)-THF to a subject in need of such treatment.

Another embodiment is directed at the reconstituted pharmaceutical compositions for use in the treatment of cancer, in particular a cancer form selected from colon cancer, stomach cancer, breast cancer, bowel cancer, gallbladder cancer, lung cancer (specifically adenocarcinoma), colorectal cancer (CRC) including metastatic CRC, head and neck cancer, liver cancer and pancreatic cancer.

Another embodiment is directed at the use of the reconstituted pharmaceutical compositions for the manufacture of a medicament for the treatment of cancer, in particular a cancer form selected from colon cancer, stomach cancer, breast cancer, bowel cancer, gallbladder cancer, lung cancer (specifically adenocarcinoma), colorectal cancer (CRC) including metastatic CRC, head and neck cancer, liver cancer and pancreatic cancer.

In another embodiment, reconstituted pharmaceutical compositions are used in therapy, preferably in chemotherapy, i.e. in the treatment of cancer. Examples of cancers to be treated include, but are not limited to, breast cancer, esophageal cancer, gastric cancer, gall bladder cancer, bile duct cancer, colon cancer, rectal cancer, liver cancer, pancreatic cancer, ovarian cancer, head and neck cancer, and mesotheolioma cancer.

In a preferred embodiment the cancer is selected from various cancer forms including colon cancer, stomach cancer, breast cancer, bowel cancer, gallbladder cancer, lung cancer (specifically adenocarcinoma), colorectal cancer (CRC) including metastatic CRC, head and neck cancer, liver cancer and pancreatic cancer.

The reconstituted pharmaceutical compositions are in a form suitable for parenteral administration, such as intravenously or intramuscularly, subcutaneously, or intra-arterially.

For parenteral administration, fluid unit dosage forms typically comprise reconstituted lyophilisates, preferably reconstituted pharmaceutical compositions, optionally a further therapeutic agent, and a pharmaceutically acceptable carrier or diluent, to form e.g. water-based solutions or oil-based suspensions. For parenteral solutions, the lyophilisates of the present invention may be filter sterilized during its preparation, e.g. before filling into a suitable vial or ampoule.

In case of a combination therapy of reconstituted pharmaceutical composition and at least one further therapeutic agent, the active agents may be administered as part of the same pharmaceutical composition or the at least one further therapeutic agent may be administered separately, i.e. as a separate (and possibly different) pharmaceutical compositions, optionally via different administration routes, either simultaneously or sequentially.

The dose of the active agent(s), i.e. 5,10-CH₂-(6R)-THF (and optionally the at least one further therapeutic agent), used in a treatment as described herein, will depend on various factors, including age and health condition of the subject to be treated, type and severity of the disease to be treated, and frequency of administration, and the like. Those skilled in the art of cancer treatment and chemotherapy would be able to determine therapeutically effective amounts and regimens for the active pharmaceutical ingredient 5,10-CH₂-(6R)-THF alone or in combination with at least one further therapeutic agent as defined above, based on known protocols for evaluating toxicity and efficacy.

The term "therapeutically effective amount" refers to the amount of active compound that elicits the biological or medicinal response in a tissue, system, animal, individual or human that is being sought by a skilled practitioner (e.g. researcher, veterinarian, medical doctor or other clinician or caregiver), which includes (i) prevention of the disease; and/or (ii) inhibition of the disease (e.g. arresting further development of the pathology and/or symptomatology); and/or (iii) amelioration of the disease (e.g. reversing the pathology and/or symptomatology). Likewise, the term "treatment" as used herein refers to (i) prevention of the disease; and/or (ii) inhibition of the disease (e.g. arresting further development of the pathology and/or symptomatology); and/or (iii) amelioration of the disease (e.g. reversing the pathology and/or symptomatology).

A pharmaceutical composition of choice may contain from 0.1% to 99 wt%, preferably from 10 to 60 wt%, of the active pharmaceutical ingredient (i.e. 5,10-CH₂-(6R)-THF optionally in combination with at least one further therapeutic agent), depending on the method of administration.

Typical dosage ranges of the 5,10-CH₂-(6R)-THF to be used in cancer treatment may range from 5 mg/m² to 1.5 g/m², preferably from 30 mg/m² to 500 mg/m² (for colorectal cancer treatment) resp. 10 mg/m² to 1000 mg/m² (for Methotrexate therapy), and more preferably from about 60 mg/m²to about 300 mg/m² (for colorectal cancer treatment) resp. 50 mg/m² to 500 mg/m² (for Methotrexate therapy).

The term "purity," as used herein, means percentage of a particular compound in a sample. The term "substantially pure", as used herein, refers to a compound in about 80% purity, preferably about 90%, more preferably about 95%, more preferably about 97%, more preferably about 98% purity, and most preferably 99% or higher than 99%, e.g., 99.5, 99.6, 99.7, 99.8, 99.9 or up to 100% purity, as determined by HPLC. Impurities may include unreacted starting material and solvents, degradation products of 5,10-CH₂-(6R)-THF (such as 5,10-methylene-(6R)-tetrahydropteroic acid and other degradation products), etc..

The term "pharmaceutically acceptable" as used herein indicates that the carrier is approved or recognized for use in animals, and more particularly in humans, i.e. it is not toxic to the host or patient. In addition, a carrier of choice will not interfere with the effectiveness of the biological activity of the active ingredient. The term "carrier" refers to any auxiliary material necessary for the particular mode of administration of choice and includes e.g. solvents (diluents) excipients, or other additives with which the lyophilisates of the present invention is administered. Typically used diluents pharmaceutical carriers include sterile liquids, such as aqueous solutions and oils (e.g. of petroleum, animal, vegetable or synthetic origin), e.g. peanut oil, soybean oil, mineral oil, sesame oil and the like. Typically used aqueous liquids include water, saline solutions, aqueous dextrose and glycerol solutions and the like. Suitable pharmaceutical excipients include starch, glucose, lactose, sucrose, gelatine, malt, rice, flour, chalk, silica gel, sodium stearate, glycerol monostearate, talc, dried skim milk, glycerol, propylene, glycol, water, ethanol and the like. Examples of suitable pharmaceutical carriers are well known in the art and are described in e.g. "Remington's Pharmaceutical Sciences" by E.W. Martin (18th ed., Mack Publishing Co., Easton, PA (1990)).

Throughout this specification and the claims which follow, unless the context requires otherwise, the word "comprise", and variations such as "comprises" or "comprising", will be understood to imply the inclusion of a stated compound, or class or group of compounds, or group of steps but not the exclusion of any other integer or step or group of integers or steps.

### BRIEF DESCRIPTION OF THE FIGURES

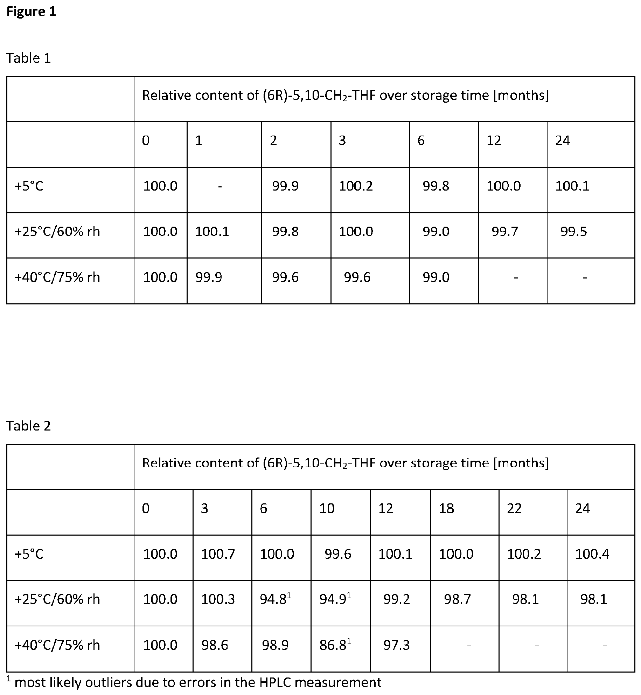

**Figure 1** contains two tables: Table 1 (top) and Table 2 (bottom).
   Table 1 demonstrates the long-term stability of a lyophilisate containing 5,10-methylene-(6R)-tetrahydrofolic acid and NaCl as prepared according to Example 2. Table 2 similarly demonstrates the long-term stability of a lyophilisate containing 5,10-methylene-(6R)-tetrahydrofolic acid and NaCl as prepared according to Example 3.

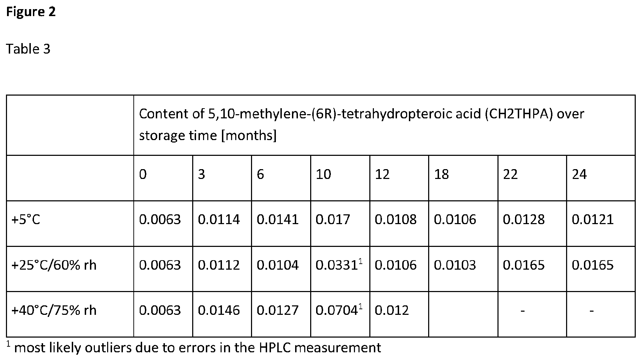

**Figure 2** contains Table 3
   Table 3 demonstrates the long-term stability of a lyophilisate containing 5,10-methylene-(6R)-tetrahydrofolic acid and NaCl as prepared according to Example 2 by following the development of the content of one of the main degradation products, 5,10-methylene-(6R)-tetrahydropteroic acid, in a lyophilisate containing 5,10-methylene-(6R)-tetrahydrofolic acid and NaCl as prepared according to Example 3.

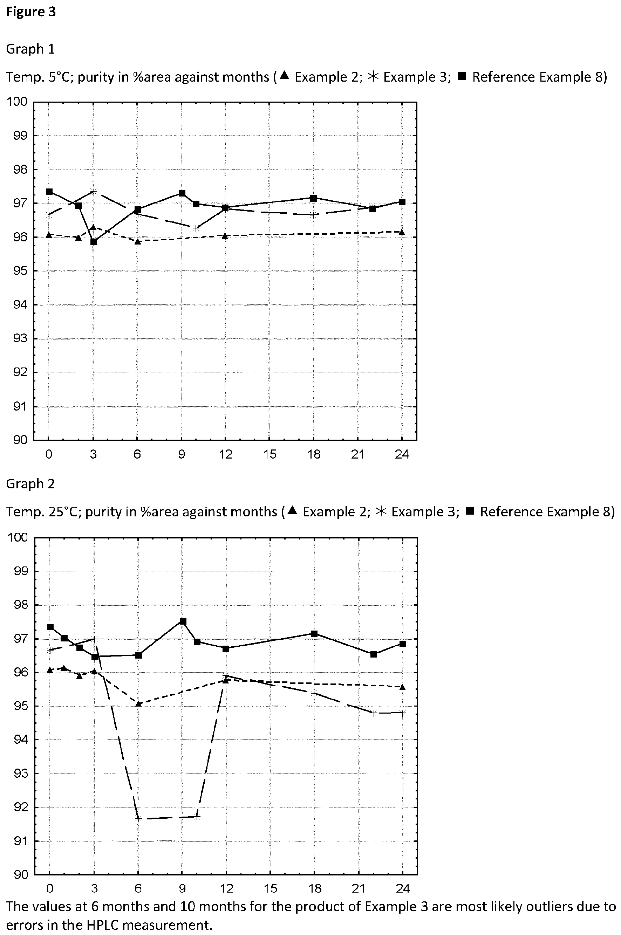

**Figure 3** contains two scatterplots: Graph 1 (top) and Graph 2 (bottom)
   The figure contains graphs which compare the effect of NaCl *vs.* citrate on the stability of the lyophilisates at different storage temperatures. As witnessed by the development of the content of 5,10-methylene-(6R)-tetrahydrofolic acid over time, the effect of NaCl is comparable to that of citrate. Graph 1 shows the long-term stability of lyophilisates containing 5,10-methylene-(6R)-tetrahydrofolic acid and NaCl as prepared according to Example 2, Example 3 and Reference Example 8 at 5°C. Graph 2 similarly shows the long-term stability of lyophilisates containing 5,10-methylene-(6R)-tetrahydrofolic acid and NaCl as prepared according to Example 2, Example 3 and Reference Example 8 at 25°C.

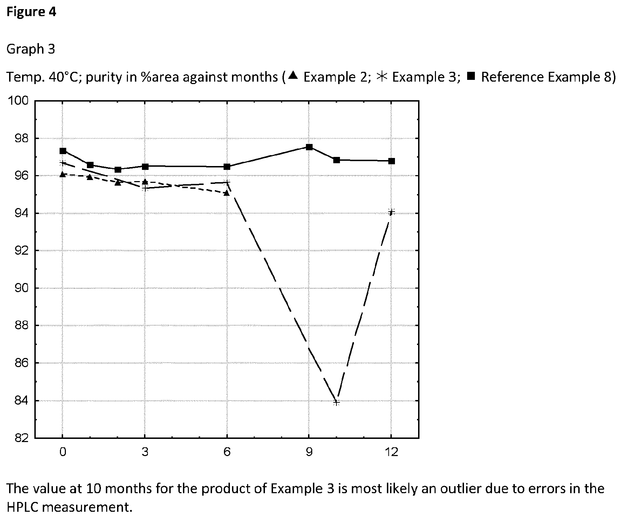

**Figure 4** contains one scatterplot: Graph 3
   The figure contains a graph which compares the effect of NaCl *vs.* citrate on the stability of the lyophilisates at an elevated storage temperature. As witnessed by the development of the content of 5,10-methylene-(6R)-tetrahydrofolic acid over time, the effect of NaCl is comparable to that of citrate. Graph 3 shows the long-term stability of lyophilisates containing 5,10-methylene-(6R)-tetrahydrofolic acid and NaCl as prepared according to Example 2, Example 3 and Reference Example 8 at 40°C.

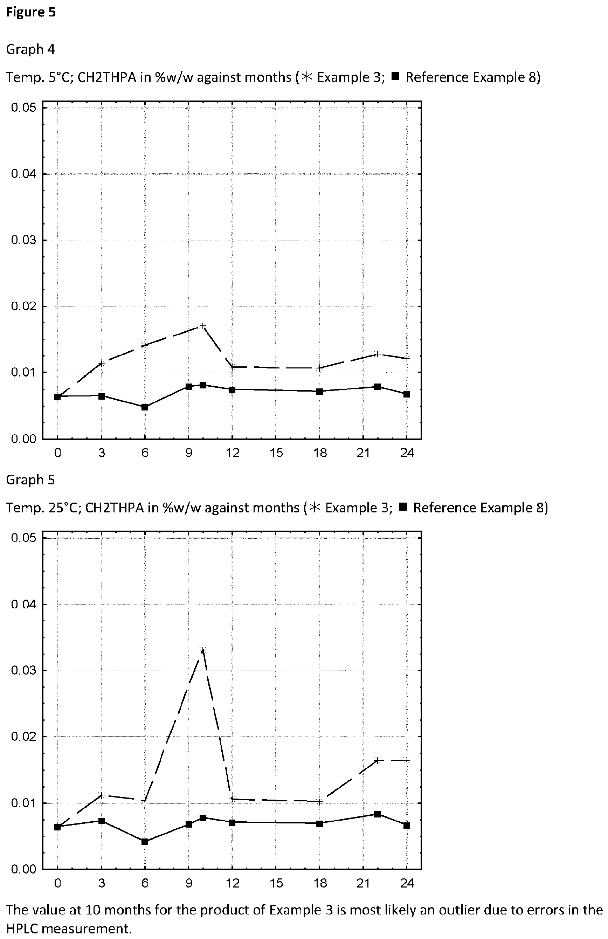

**Figure 5** contains two scatterplots: Graph 4 (top) and Graph 5 (bottom)
   The figure contains graphs which compare the effect of NaCl *vs.* citrate on the stability of the lyophilisates at different storage temperatures. As witnessed by the development of the content of one of the main degradation products, 5,10-methylene-(6R)-tetrahydropteroic acid (CH2THPA), over time, the effect of NaCl is comparable to that of citrate.
   Graph 4 shows the development of the content of one of the main degradation products, 5,10-methylene-(6R)-tetrahydropteroic acid (CH2THPA), in lyophilisates containing 5,10-methylene-(6R)-tetrahydrofolic acid and NaCl as prepared according to Example 3 and Reference Example 8 at 5°C.
   Graph 5 similarly shows the development of the content of the one of the main degradation products, 5,10-methylene-(6R)-tetrahydropteroic acid (CH2THPA), in lyophilisates containing 5,10-methylene-(6R)-tetrahydrofolic acid and NaCl as prepared according to Example 3 and Reference Example 8 at 25°C.

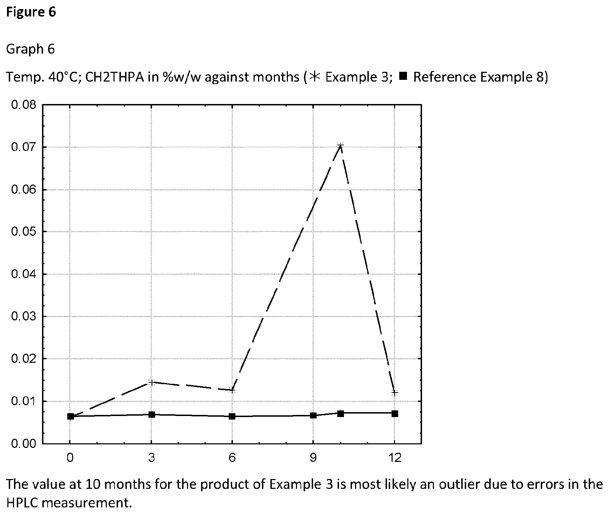

**Figure 6** contains one scatterplot: Graph 6
   The figure contains a graph which compares the effect of NaCl *vs.* citrate on the stability of the lyophilisates at an elevated storage temperature. As witnessed by the development of the content of one of the main degradation products, 5,10-methylene-(6R)-tetrahydropteroic acid (CH2THPA), over time, the effect of NaCl is comparable to that of citrate.
   Graph 6 shows the development of the content of one of the main degradation products, 5,10-methylene-(6R)-tetrahydropteroic acid (CH2THPA), in lyophilisates containing 5,10-methylene-(6R)-tetrahydrofolic acid and NaCl as prepared according to Example 3 and Reference Example 8 at 40°C.

### EXAMPLES

### HPLC

For the measurement of purity/content and degradation products an HPLC-UV Gradient Method was used: Column type: ODS, Mobile phase: A: aqueous Buffer; Mobile Phase: B: aqueous Buffer/Methanol, Run time: 30min, Sample Solvent: aqueous Buffer

### Water content

The determination of the water content was made according to Ph. Eur. 2.5.32/USP <921/ Method Ic>

### Osmolality

The determination of the osmolality was made according to Ph. Eur. 2.2.35 (osmometer)/USP <785>

### Example 1: Lyophilisate containing 5,10-methylene-(6R)-tetrahydrofolic acid and NaCl

Under nitrogen 210 g purified water and 16.5 g sodium hydroxide 2M were cooled down to 3±2°C (resulting pH 14.0). 4.57 g NaCl and then 5.70 g 5,10-methylene-(6R)-tetrahydrofolic acid hemisulfate salt were added (pH is starting to decrease) and rinsed with 2.5 g purified water. By the addition of 2M sodium hydroxide, pH was held at 9.3±0.1. 18.74 g purified water was added. Overall, 20.8 g 2M sodium hydroxide was needed to keep the pH at 9.3±0.1.

5.0 ml per vial of the resulting clear solution was transferred into 10 ml glass vials (36 vials). Vials were immediately frozen with liquid nitrogen and lyophilised at < 10⁻¹ mbar.

Vials obtained contained a lyophilisate of 102 mg 5,10-methylene-(6R)-tetrahydrofolic acid (calculated as free acid) with NaCl whereby w/w percentage of 5,10-methylene-(6R)-tetrahydrofolic acid in the lyophilisate is 40% of the total solids in lyophilisate.

When reconstituted the solution has an osmolality of 346 mOsmol/kg.

### Example 2: Lyophilisate containing 5,10-methylene-(6R)-tetrahydrofolic acid and NaCl

Under nitrogen 210 g purified water and 16.5 g sodium hydroxide 2M were cooled to 3±2°C (resulting pH 14.0). 4.10 g NaCl and 5.70 g 5,10-methylene-(6R)-tetrahydrofolic acid hemisulfate salt were added (pH is staring to decrease) and rinsed with 2.5 g purified water. By the addition of 2M sodium hydroxide pH is held at 9.3±0.1. 18.8 g purified water was added. Overall, 21.2 g 2M sodium hydroxide was needed to keep the pH at 9.3±0.1.

5.0 ml of the resulting clear solution was transferred per vial into 10 ml glass vials (36 vials). Vials were immediately frozen with liquid nitrogen and lyophilised at < 10⁻¹ mbar.

Vials obtained contained a lyophilisate of 102 mg 5,10-methylene-(6R)-tetrahydrofolic acid (calculated as free acid) with NaCl. 5,10-methylene-(6R)-tetrahydrofolic acid is showing a purity of 93.2% w/w (as free acid) measured by HPLC.

When reconstituted the solution has an osmolality of 320 mOsmol/kg.

### Example 3: Lyophilisate containing 5,10-methylene-(6R)-tetrahydrofolic acid and NaCl

Under nitrogen 210 g purified water and 16.5 g sodium hydroxide 2M were cooled to 3±2°C (resulting pH 14.0). 4.10 g NaCl and 5.80 g 5,10-methylene-(6R)-tetrahydrofolic acid hemisulfate salt were added (pH is starting to decrease) and rinsed with 2.5 g purified water. By the addition of 2M sodium hydroxide pH is held at 9.3±0.1. 18.8 g purified water was added. Overall, 21.2 g 2M sodium hydroxide was needed to keep the pH at 9.3±0.1.

5.0 ml per vial of the resulting clear solution was transferred into 10 ml glass vials (36 vials). Vials were immediately frozen with liquid nitrogen and lyophilised at < 10⁻¹ mbar.

Vials obtained contained a lyophilisate of 97.468 mg 5,10-methylene-(6R)-tetrahydrofolic acid (calculated as free acid) with NaCl. 5,10-methylene-(6R)-tetrahydrofolic acid is showing a purity of 93.2% w/w (as free acid) resp. 96.7% area, and the sum of by-products was 1.07%, both measured by HPLC. Water content was 0.63% w/w.

When reconstituted the solution has an osmolality of 325 mOsmol/kg and a pH of 8.23.

### Example 4: Lyophilisate containing 5,10-methylene-(6R)-tetrahydrofolic acid and NaCl

Under nitrogen 210 g purified water and 16.5 g sodium hydroxide 2M were held at 25±2°C (resulting pH 13.2). 4.10 g NaCl and 5.80 g 5,10-methylene-(6R)-tetrahydrofolic acid hemisulfate salt were added (pH is starting to decrease) and rinsed with 2.5 g purified water. By the addition of 2M sodium hydroxide pH is held at 9.3±0.1. 17.1 g purified water were added. Overall, 21.2 g 2M sodium hydroxide was needed to keep the pH at 9.3±0.1.

5.0 ml per vial of the resulting clear solution was transferred into 10 ml glass vials (36 vials). Vials were immediately frozen with liquid nitrogen and lyophilised at < 10⁻¹ mbar.

Vials obtained contained a lyophilisate of 5,10-methylene-(6R)-tetrahydrofolic acid with NaCl, showing for 5,10-methylene-(6R)-tetrahydrofolic acid a purity of 95.9% area and a sum of by-products of 1.07%, both measured by HPLC. Water content was 0.67% w/w.

When reconstituted the solution has an osmolality of 329 mOsmol/kg and a pH of 9.06.

### Example 5: Long term stability of lyophilisates containing 5,10-methylene-(6R)-tetrahydrofolic acid and NaCl (content of (6R)-5,10-CH₂-THF)

In order to determine the long-term stabilities of lyophilisates of 5,10-CH₂-(6R)-THF prepared according to Examples 1-4, lyophilisates were stored in air at +5°C, +25°C/60% relative humidity and +40°C/75% relative humidity. The content of 5,10-CH₂-(6R)-THF remaining was measured by HPLC at periodic intervals and is given by comparison with the initial value (% rel.). The results are shown in Tables 1 and 2 (Graph 1-3).

Tables 1 and 2 (Graph 1-3) clearly show that lyophilisates of 5,10-CH₂-(6R)-THF with NaCl are highly stable over a long period of time even at elevated temperatures.

### Example 6: Long term stability of lyophilisates containing 5,10-methylene-(6R)-tetrahydrofolic acid and NaCl (content of stability indicator 5,10-methylene-(6R)-tetrahydropteroic acid)

In order to determine the long-term stabilities of lyophilisates of (6R)-5,10-CH₂-THF prepared according to Examples 1 -4, lyophilisates were stored in air at +5°C, +25°C/60% relative humidity and +40°C/75% relative humidity. The content of one of the main degradation product 5,10-methylene-(6R)-tetrahydropteroic acid (CH2THPA) was measured by HPLC at periodic intervals. The results are shown in Table 3 (Graph 4-6).

Table 3 (Graph 4-6) confirm that lyophilisates of 5,10-CH₂-(6R)-THF and NaCl are highly stable over a long period of time even at elevated temperatures, also as witnessed by the very slow increase in content of one of the main degradation product 5,10-methylene-(6R)-tetrahydropteroic acid (CH2THPA).

### Example 7: Reconstitution of lyophilisates containing 5,10-methylene-(6R)-tetrahydrofolic acid and NaCl

Lyophilisates as prepared according to Examples 1 to 4 can easily be reconstituted by adding 10 ml water for injection to the vial. The lyophilisate dissolves completely.

### Reference Example 8: Composition containing 5,10-methylene-(6R)-tetrahydrofolic acid and citrate, having adjusted the pH to a basic value (EP 1 641 460)

As reference a composition containing 5,10-CH₂-(6R)-THF and citrate having adjusted the pH to 8.5 - 9.5 as disclosed in EP 1 641460 was prepared according to the following procedure.

Under nitrogen 210 g purified water and 16.5 g sodium hydroxide 2M were held at 3±2°C (resulting pH 13.9). 11.50 g tri-sodium citrate and 5.80 g 5,10-methylene-(6R)-tetrahydrofolic acid hemisulfate salt were added (pH is starting to decrease) and rinsed with 2.5 g purified water. By the addition of 2M sodium hydroxide pH is held at 9.3±0.1. 11.0 g purified water were added. Overall, 13.8 g 2M sodium hydroxide was needed to keep the pH at 9.3±0.1.

5.0 ml per vial of the resulting clear solution was transferred into 10 ml glass vials (36 vials). Vials were immediately frozen with liquid nitrogen and lyophilised at < 10⁻¹ mbar.

Vials obtained contained a lyophilisate of 5,10-methylene-(6R)-tetrahydrofolic acid with citrate, showing for 5,10-methylene-(6R)-tetrahydrofolic acid a purity of 97.8% area and a sum of by-products of 0.81%, both measured by HPLC.

## Claims

1. Stable lyophilisate comprising 5,10-methylene-(6R)-tetrahydrofolic acid and NaCl, wherein the molar ratio of 5,10-methylene-(6R)-tetrahydrofolic acid to NaCl is from 1:1 to 1:10.

2. Stable lyophilisate according to claim 1, further **characterized in** comprising no further chemotherapeutic agents.

3. Stable lyophilisate according to claim 1 or 2, wherein the molar ratio of 5,10-methylene-(6R)-tetrahydrofolic acid to NaCl is from 1:5 to 1:10.

4. Stable lyophilisate according to any one of claim 1 to 3, wherein the molar ratio of 5,10-methylene-(6R)-tetrahydrofolic acid to NaCl is 1:7.

5. Stable lyophilisate according to any one of claim 1 to 4, wherein the employed 5,10-methylene-(6R)-tetrahydrofolic acid has a purity of greater than 99%, such as a purity of greater than 99.5%.

6. Stable lyophilisate according to any one of claims 1 to 5, **characterized in** containing neither citric acid nor citrate ions.

7. Stable lyophilisate according to any of claims 1 to 6, having a water content of less than 5%.

8. Stable lyophilisate according to any of claims 1 to 6, having a water content of at most 3%.

9. Stable lyophilisate according to any of claims 1 to 6, having a water content below 1%.

10. Stable lyophilisate according to any of claims 1 to 6, being anhydrous.

11. Stable lyophilisate according to any one of claims 1 to 10, further comprising one or more pharmaceutically acceptable excipients and/or osmolality correcting excipients and/or matrix stabilisers.

12. Stable lyophilisate according to any one of claims 1 to 11, further comprising a buffer.

13. Process for preparing a stable lyophilisate according to any one of claims 1 to 12 comprising the steps of
(i) dissolving 5,10-methylene-(6R)-tetrahydrofolic acid, or a salt thereof, in a solvent or solvent system,
(ii) adding NaCl,
(iii) freezing the solution, and
(iv) thereafter removing the frozen solvent under vacuum,
or
(i) dissolving 5,10-methylene-(6R)-tetrahydrofolic acid, or a salt thereof, in a solvent or solvent system,
(iia) adding NaOH to raise the pH to about 11,
(iib) adding HCl to lower the pH to about 9.3,
(iii) freezing the solution, and
(iv) thereafter removing the frozen solvent under vacuum.

14. Process according to claim 13, wherein the solvent is water or an aqueous solvent system.

15. Process according to claim 13 or 14, wherein in step (ii), (iia) and/or (iib) osmolality correcting excipients and/or matrix stabilisers are added.

## Patentansprüche

1. Stabiles Lyophilisat, umfassend 5,10-Methylen-(6R)-tetrahydrofolsäure und NaCl, wobei das Molverhältnis von 5,10-Methylen-(6R)-tetrahydrofolsäure zu NaCl zwischen 1:1 und 1:10 liegt.

2. Stabiles Lyophilisat gemäß Anspruch 1, das ferner **dadurch gekennzeichnet ist, dass** es keine weiteren Chemotherapeutika umfasst.

3. Stabiles Lyophilisat gemäß Anspruch 1 oder 2, wobei das Molverhältnis von 5,10-Methylen-(6R)-tetrahydrofolsäure zu NaCl zwischen 1:5 und 1:10 liegt.

4. Stabiles Lyophilisat gemäß einem der Ansprüche 1 bis 3, wobei das Molverhältnis von 5,10-Methylen-(6R)-tetrahydrofolsäure zu NaCl 1:7 beträgt.

5. Stabiles Lyophilisat gemäß einem der Ansprüche 1 bis 4, wobei die verwendete 5,10-Methylen-(6R)-tetrahydrofolsäure eine Reinheit von mehr als 99 % aufweist, wie beispielsweise eine Reinheit von mehr als 99,5 %.

6. Stabiles Lyophilisat gemäß einem der Ansprüche 1 bis 5, **dadurch gekennzeichnet, dass** es weder Zitronensäure noch Citrat-Ionen enthält.

7. Stabiles Lyophilisat gemäß einem der Ansprüche 1 bis 6, das einen Wassergehalt von weniger als 5 % aufweist.

8. Stabiles Lyophilisat gemäß einem der Ansprüche 1 bis 6, das einen Wassergehalt von höchstens 3 % aufweist.

9. Stabiles Lyophilisat gemäß einem der Ansprüche 1 bis 6, das einen Wassergehalt von weniger als 1 % aufweist.

10. Stabiles Lyophilisat gemäß einem der Ansprüche 1 bis 6, das wasserfrei ist.

11. Stabiles Lyophilisat gemäß einem der Ansprüche 1 bis 10, das ferner einen oder mehrere pharmazeutisch verträgliche Hilfsstoffe und/oder osmolalitätskorrigierende Hilfsstoffe und/oder Matrixstabilisatoren umfasst.

12. Stabiles Lyophilisat gemäß einem der Ansprüche 1 bis 11, das ferner einen Puffer umfasst.

13. Verfahren zur Herstellung eines stabilen Lyophilisats gemäß einem der Ansprüche 1 bis 12, umfassend die folgenden Schritte:
(i) Auflösen von 5,10-Methylen-(6R)-tetrahydrofolsäure oder eines Salzes davon in einem Lösungsmittel oder Lösungsmittelsystem,
(ii) Zugeben von NaCl,
(iii) Einfrieren der Lösung und
(iv) anschließendes Entfernen des gefrorenen Lösungsmittels unter Vakuum,
oder
(i) Auflösen von 5,10-Methylen-(6R)-tetrahydrofolsäure oder eines Salzes davon in einem Lösungsmittel oder Lösungsmittelsystem,
(iia) Zugeben von NaOH, um den pH-Wert auf etwa 11 anzuheben,
(iib) Zugeben von HCl, um den pH-Wert auf etwa 9,3 zu senken,
(iii) Einfrieren der Lösung und
(iv) anschließendes Entfernen des gefrorenen Lösungsmittels unter Vakuum.

14. Verfahren nach Anspruch 13, wobei das Lösungsmittel Wasser oder ein wässriges Lösungsmittelsystem ist.

15. Verfahren nach Anspruch 13 oder 14, wobei in den Schritten (ii), (iia) und/oder (iib) osmolalitätskorrigierende Hilfsstoffe und/oder Matrixstabilisatoren zugegeben werden.

## Revendications

1. Lyophilisat stable comprenant de l'acide 5,10-méthylène-(6R)-tétrahydrofolique et du NaCl, dans lequel le rapport molaire de l'acide 5,10-méthylène-(6R)-tétrahydrofolique au NaCl est compris entre 1:1 et 1:10.

2. Lyophilisat stable selon la revendication 1, **caractérisé en ce qu'**il ne comprend aucun autre agent chimiothérapeutique.

3. Lyophilisat stable selon la revendication 1 ou 2, dans lequel le rapport molaire de l'acide 5,10-méthylène-(6R)-tétrahydrofolique au NaCl est compris entre 1:5 et 1:10.

4. Lyophilisat stable selon l'une quelconque des revendications 1 à 3, dans lequel le rapport molaire de l'acide 5,10-méthylène-(6R)-tétrahydrofolique au NaCl est de 1:7.

5. Lyophilisat stable selon l'une quelconque des revendications 1 à 4, dans lequel l'acide 5,10-méthylène-(6R)-tétrahydrofolique utilisé présente une pureté supérieure à 99 %, par exemple une pureté supérieure à 99,5 %.

6. Lyophilisat stable selon l'une quelconque des revendications 1 à 5, **caractérisé en ce qu'**il ne contient ni acide citrique ni ions citrate.

7. Lyophilisat stable selon l'une quelconque des revendications 1 à 6, présentant une teneur en eau inférieure à 5 %.

8. Lyophilisat stable selon l'une quelconque des revendications 1 à 6, présentant une teneur en eau inférieure ou égale à 3 %.

9. Lyophilisat stable selon l'une quelconque des revendications 1 à 6, présentant une teneur en eau inférieure à 1 %.

10. Lyophilisat stable selon l'une quelconque des revendications 1 à 6, qui est anhydre.

11. Lyophilisat stable selon l'une quelconque des revendications 1 à 10, comprenant en outre un ou plusieurs excipients pharmaceutiquement acceptables et/ou des excipients correcteurs d'osmolalité et/ou des stabilisants de matrice.

12. Lyophilisat stable selon l'une quelconque des revendications 1 à 11, comprenant en outre un tampon.

13. Procédé de préparation d'un lyophilisat stable selon l'une quelconque des revendications 1 à 12, comprenant les étapes consistant à
(i) dissoudre l'acide 5,10-méthylène-(6R)-tétrahydrofolique, ou un de ses sels, dans un solvant ou un système de solvants,
(ii) ajouter du NaCl,
(iii) congeler la solution, et
(iv) éliminer ensuite le solvant congelé sous vide,
ou
(i) dissoudre l'acide 5,10-méthylène-(6R)-tétrahydrofolique, ou un de ses sels, dans un solvant ou un système de solvants,
(iia) ajouter du NaOH pour porter le pH à environ 11,
(iib) ajouter du HCl pour abaisser le pH à environ 9,3,
(iii) congeler la solution, et
(iv) éliminer ensuite le solvant congelé sous vide.

14. Procédé selon la revendication 13, dans lequel le solvant est de l'eau ou un système de solvants aqueux.

15. Procédé selon la revendication 13 ou 14, dans lequel, à l'étape (ii), (iia) et/ou (iib), des excipients correcteurs d'osmolalité et/ou des stabilisants de matrice sont ajoutés.
